# EUROPEAN PATENT SPECIFICATION

(11) **EP 3 875 048 B1**
(45) Date of publication and mention of the grant of the patent: **04.05.2022**
(21) Application number: 20161010.2
(22) Date of filing: 04.03.2020
(51) Int. Cl.: A61B 17/225, A61B 8/08, A61B 34/20, A61G 13/02, A61B 6/12, A61B 6/00, A61B 8/00, A61B 34/00, A61B 90/50, A61G 13/04, A61B 17/00, A61B 90/00

(54) **SHOCKWAVE THERAPY SYSTEM WITH 3D CONTROL**
STOSSWELLENTHERAPIESYSTEM MIT 3D-STEUERUNG
SYSTÈME DE THÉRAPIE D'ONDES DE CHOC AVEC CONTRÔLE 3D

(43) Date of publication of application: 08.09.2021
(73) Proprietor: Storz Medical AG, 8274 Tägerwilen (CH)
(72) Inventor: Gleibe, Olaf, 8280 Kreuzlingen (CH); Musterle, Jochem, 78464 Konstanz (DE); Knödgen, Fritz, 78464 Konstanz (DE); Müller, Peter, 8583 Sulgen (CH)
(74) Representative: Lohr, Jöstingmeier & Partner

(56) References cited:
- EP-A2- 3 047 809
- US-A1- 2016 038 166
- US-A1- 2018 243 151
- US-B1- 6 773 408
- US-B2- 9 815 206
- US-B2- 10 524 812

## Description

### Field of the invention

The invention relates to an extracorporeal shock wave therapy system, e.g. a lithotripsy system for non-invasive treatment of stones, like e.g. kidney stones, urinary stones, gallstones or other calculi within a mammal's body using acoustic pulses.

### Description of the related art

A device and a method for localizing stones and for targeting a shock wave source of a lithotripter is disclosed in DE 198 41 951 A1. It uses X-ray imaging and generates a pseudo 3D image on a screen to simplify targeting. To avoid x-ray exposure, in-line ultrasound systems have been developed. Here, a diagnostic ultrasound transducer is arranged in a line with the therapeutic shock wave source. An off-line ultrasound localization, using a handheld, freely positionable ultrasound transducer is more flexible and allows better images. A further targeting method is disclosed in EP 2 340 781 A1. Here a shockwave generator, a patient table and an ultrasound targeting device are provided with optical markers for tracking with a pair of infrared cameras. The precision and reliability of the tracking system determine the total system precision. A verification may be done by using an X-ray system.

EP 3 047 809 A2 discloses a lithotripter having a patient table which is movable in multiple axes.

US 9,815,206 B2 discloses a surgical robot controlled by a 3D mouse.

US 2018/0243151 A1 discloses a surgical robot with a five axes control unit.

US 6,773,408 B1 discloses an ultrasound applicator which may be controlled by an MRA.

US 2016/0038166 A1 discloses a simple lithotripter.

US 10,524,812 B2 discloses a lithotripter with optical positioning.

### Summary of the invention

The problem to be solved by the invention is to provide a control means for controlling the movement and/or position of the shock wave source relative to the table. Positioning should be easy and quick.

Solutions of the problem are described in the independent claim 1. The dependent claims relate to further improvements of the invention.

The embodiments relate to an ultrasound and/or shockwave therapy system for non-invasive treatment of the human and/or animal body. Such a therapy system may be an ultrasound and/or shockwave lithotripsy system. It may also be a shockwave system for cardiac treatment. An ultrasound and/or shockwave therapy system includes an ultrasound and/or shockwave device which further includes at least one ultrasound and/or shockwave source. Herein, the ultrasound and/or shockwave source may simply be mentioned as source. The ultrasound and/or shockwave source is configured to generate a strong ultrasound signal or shockwaves or both. The ultrasound signal and/or the shockwaves may be focused to a focal spot. Furthermore, a patient table is provided for accommodating a patient. A positioning device is provided between the ultrasound/shockwave source and the patient table configured to modify the spatial relationship between the ultrasound and/or shockwave source and the patient table. The positioning device may also be configured to modify the orientation of the ultrasound and/or shockwave source relative to the patient table. The positioning device may be configured for a relative movement in at least five degrees of freedom. The positioning device may allow a three-axis positioning, like a positioning in an x-axis corresponding to a longitudinal axis of the table, a positioning a y-axis which is a transverse axis to the patient table, and along the z-axis which is oriented perpendicular to the table surface. The x-, y-, and z-axes may form a Cartesian coordinate system. A three-axis movement or translation may allow to bring a focus spot of an ultrasound and/or shockwave source of the ultrasound and/or shockwave device in close proximity to or on the target area. This target area may include a stone to be disintegrated or a specific area of a heart. To avoid unwanted damage of organs or other body parts, it may be useful to couple the ultrasound and/or shockwave energy under an angle to the target area. This may require tilting of the ultrasound and/or shockwave source relative to the body. Therefore, a tilting about one or preferably two axis/axes may be provided. The cartesian coordinate system may be defined by the table, where the surface of table defines the x- and y-axis plane.

The positioning device may have 3 cartesian linear displacement axes and 2 tilting axes in the plane of the table. In another embodiment, the positioning device may have a differing arrangement. An example is, where the plane of the tilting axes is not parallel to the table plane. I such a case the positioning device may still make a tilting in a plane of the table by making at the same time a linear displacement to compensate for the non-parallel plane.

There may be therapy systems which have a stationary patient table and where the ultrasound and/or shockwave source is moved as required. Alternatively, there may be a combined movement of the ultrasound and/or shockwave source and of the patient table. For example, the patient table may be moved along an x-axis and/or a y-axis, whereas the other movements are made by the ultrasound and/or shockwave source. There may be other movements, for example a movement along an x-axis of the table and a tilting of the table in the x/y-plane. In general, such movements can also be described as movements in a Cartesian coordinate system's x, y, z axes. Positioning of the ultrasound and/or shockwave source with respect to the target area may be controlled by a computer which may receive input from a keyboard and/or a touch screen, where virtual and/or mechanical buttons or switches may be provided to control displacement and/or tilting. Manual control of individual movements, e.g. like translation along axes or tilting is difficult and time-consuming.

As the patient and therefore a target area, which may be a stone to be disintegrated within the body of the patient may be positioned on the table in a stabilized manner, such that neither the patient nor the target area, which may include a stone, moves relative to the table, a positioning of the ultrasound and/or shockwave source relative to the table would result in a positioning relative to the patient and relative to the target area . Therefore, by a positioning of the ultrasound and/or shockwave source relative to the patient table, targeting of the focus spot of the ultrasound and/or shockwave source with respect to the target area may be achieved.

It should be noted that herein, reference is made to the displacement of the ultrasound and/or shockwave source. The ultrasound and/or shockwave source is part of the ultrasound and/or shockwave device. To move the ultrasound and/or shockwave source, the ultrasound and/or shockwave source itself may be moved. Alternatively or additionally the ultrasound and/or shockwave device containing the source may be moved.

A control device may be provided, which may be configured to be touched or moved by an operator and which may be configured to sense forces and/or movements in at least five degrees of freedom. This control device may be configured to generate output signals according to the sensed forces or movements. The at least five degrees of freedom may include within a cartesian coordinate system three degrees of translation on three orthogonal axes and at least two degrees of rotation or tilting. The control device may be attached to or integrated into one of: the patient table, the control unit, a targeting device, a display, a keyboard, a console.

A control unit may be coupled to receive the output signals from the control device and to control the positioning device to modify the spatial relationship and orientation between the source and the patient table according to the signals from the input device. The control unit may be configured to set the positioning speed of the positioning device according to the output signals of the control device based on a linear function or based on an exponential function.

In another embodiment, the control device is configured to sense forces and/or movements in six degrees of freedom. The control unit may generate control signals to the positioning device to move the source along a center axis of the source. The sixth degrees of freedom of the control device may be a force and/or movement perpendicular to the surface of control panel to which the control device is attached.

In an embodiment, a first cartesian coordinate system may be defined by a first x-axis being a longitudinal axis through the center of a surface of the patient table, a first y-axis orthogonal to the first x-axis on the surface of the patient table, and a first z-axis orthogonal to the first x-axis and the first y-axis pointing upward from the surface of the patient table.

A second cartesian coordinate system may be defined by a second x-axis being a longitudinal axis parallel to a baseplane of the control device, a second y-axis orthogonal to the second x-axis and parallel to the baseplane, and a second z-axis orthogonal to the second x-axis and the second y-axis pointing upward from the baseplane and preferably through a center axis of the control device.

The control unit may be configured to give control signals to the positioning device dependent of forces and/or movements of the control device. Such forces and/or movements of the control device along an x-axis may result in control signals for relative movement along an x-axis. Forces and/or movements of the control device along a y-axis may result in control signals for relative movement along a y-axis. Forces and/or movements of the control device along a z-axis may result in control signals for relative movement along a z-axis. Forces and/or movements of the control device for a tilt around the x-axis may result in control signals for tilt around the x-axis. Forces and/or movements of the control device for a tilt around the y-axis may result in control signals for tilt around the y-axis. The directions of relative movement may be the same as the directions of forces and/or movements of the control device. Under special circumstances they may be inverted to give the operator a better spatial feeling.

In an embodiment, a five-axis control device which may also be called a 5-axis mouse is provided to control the movement. The control device may be operated by a hand, for example of a surgeon. The control device may accept forces in a combination of different axes. It may also allow for small deflections.

If the control device is pressed and/or deflected into a positive or negative x-direction, there may be a relative movement between the source and the table in a positive or negative direction along the x-axis. This movement may be done by moving the patient table along the x-axis which is according to the definition used in this document, the longitudinal axis extending along the length of the table.

A force or deflection in either positive or negative y-direction at the control device may cause a relative movement between the source and the table in a positive or negative direction along the y-axis, which for example may be a sideward movement of the patient table, or of the ultrasound and/or shockwave source. It may also cause a rotation of the patient table in the x/y-plane with or without x-axis shifting at the same time. A rotation together with x-axis shifting may provide a linear y-axis movement.

A force and/or deflection of the control device in either positive or negative z-direction may cause a relative movement of the source device relative to the table in either direction along the z-axis.

A tilting force to or a tilting action of the control device around the x-axis may cause a tilting of the ultrasound and/or shockwave source around the x-axis. Furthermore, a tilting force to or a tilting action of the control device around the y-axis may cause a tilting of the ultrasound and/or shockwave source around the y-axis.

The functions described above provide a rather intuitive control of the movements of the ultrasound and/or shockwave source relative to the table.

If the movements of the ultrasound and/or shockwave source are assigned as described above, operation is rather intuitive as the focus spot of the ultrasound and/or shockwave source follows the movement of the control device. For example, pushing the control device along the x-axis moves the focus spot along the x-axis, or pulling the control device upwards may move the focus spot upwards. Tilting the control device around the x-axis may tilt the focus spot around the x-axis. This may result in a very simple, easy, and fast setting of the relative position between the ultrasound and/or shockwave source with respect to the patient table and therefore the patient's body and the target area.

In an embodiment, the control device is only force-sensitive, such that it actually cannot be moved or can only be moved for a very small distance which is not noticeable by a person operating the control device. Such a force-sensitive control device may define the speed and/or the distance to be moved by the force. Therefore, a higher force may result in a higher speed of movement.

In an embodiment, the control device may be at least one of moved, displaced, deflected and tilted, which gives the user a tactile feedback. It should be mentioned that the control device described herein is not comparable to a conventional two axis joystick which simply may be moved along an x- and a y-axis and which may have an optional z-axis. The control device of the embodiments described herein allows translation along an x-, a y-, and a z-axis, or at least recognizes forces along an x-, a y-, and a z-axis. In addition, it can be tilted round an x- and a y-axis, or recognizes tilting forces around an x- or a y-axis.

A six-axis control device is provided which in addition allows rotation or at least recognizes rotational forces or movement (rotation) around a z-axis.

Rotation about the z-axis is used for additional control of at least an additional function. Such a function is the movement of the focus spot towards or away from the ultrasound and/or shockwave source. This may be done by adjusting the focal width of the source and/or by performing a combined movement on at least one of the x-axis, y-axis and z-axis. By this way the source may be displaced along an axis through the center of the source.

Such a movement may for example be controlled by a rotation of the control device around the z-axis or applying torque around the z-axis. In an embodiment, a rotation in a clockwise direction may reduce the distance, which is recognized from a person rotating the button like screwing in a screw, whereas a counterclockwise rotation which may be recognized like screwing out a screw, may increase the distance of the focus spot from the ultrasound and/or shockwave source.

The control device may have a cylindrical shape. It may have a center of suspension which may be above a baseplane to which the control device is mounted. This may allow for displacement and for tilting/rotating of the control device. The control device may have any other suitable shape like a ball or cylinder with a circular recess which may help gripping. There may also be other recesses or grooves on the outer surface of the cylinder.

An example, not being part of the invention, relates to a method of controlling the relative distance between a patient table of a therapy system and an ultrasound and/or shockwave source and/or the focus spot of an ultrasound and/or shockwave source, the method comprising at least one of the steps of:
- indicating a force and/or a movement in either a positive or negative x-direction and moving the ultrasound and/or shockwave source relative to the patient table in either the negative or the positive x-direction;
- indicating a force and/or a movement in either a positive or negative y-direction and moving the ultrasound and/or shockwave source relative to the patient table in either the negative or positive y-direction;
- indicating a force and/or a movement in either a positive or negative z-direction and moving the ultrasound and/or shockwave source relative to the patient table in either the negative or positive z-direction;
- indicating tilting force and/or a movement around the x-axis; tilting the source around the x-axis;
- indicating tilting force and/or a movement around the y-axis; tilting the source around the y-axis.

In a further example, the following step may be performed:
- indicating a torque or a rotation around a z-axis and displacing the focus spot of the ultrasound and/or shockwave source relative to the ultrasound and/or shockwave source or and displacing the focus spot of the ultrasound and/or shockwave source relative to the table.

The methods may be combined together. The method steps may be performed by using a device as described herein.

### Description of Drawings

In the following the invention will be described by way of example, without limitation of the general inventive concept, on examples of embodiment with reference to the drawings.
- Figure 1: shows a first embodiment.
- Figure 2: shows a control panel in detail.
- Figure 3: shows a focal spot translation.
- Figure 4: shows a functional block diagram.
- Figure 5: shows a modified embodiment.
- Figure 6: shows a control device.
- Figure 7: shows a displaced device.
- Figure 8: shows a tilted device.
- Figure 9: shows a further embodiment.

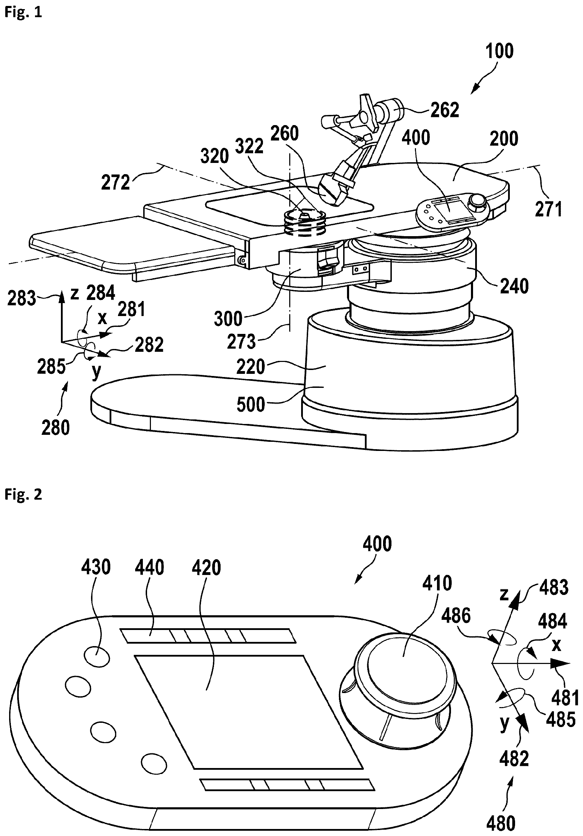

In Figure 1, a first embodiment is shown. An example of a therapy system is given. Here, a lithotripsy system is shown for example. The invention works with any ultrasound and/or shockwave treatment system.

An extra-corporal ultrasound and/or shockwave lithotripsy system for non-invasive treatment of stones 100 comprises a patient table 200 and an ultrasound and/or shockwave source 320, and a control panel 400. The table 200 has a flat surface for accommodating a patient which is not shown here. The patient may have a kidney stone which may be disintegrated by the lithotripsy system. There may be an ultrasound transducer 260, also called targeting device, for locating the stone. The area around the stone may be the target area. The ultrasound transducer 260 may be held at the table by an arm 262 and it may be movable together with the table, such that it maintains a constant spatial relationship with the table, and therefore with the patient and its stone. In another embodiment, an ultrasound transducer for locating the stone may be integrated into the source, for example arranged coaxially at the center of the source.

The patient table 200 is based on a stand 220 which may stand on a floor. At the table, there may be a positioning device 240 to move the table relative to the ultrasound and/or shockwave source 320 which may be part of the ultrasound and/or shockwave device 300. The ultrasound and/or shockwave source 320 and/or the patient table 200 may be movable to achieve a relative movement. Both parts may be movable or one of these parts may be stationary and the other one may be movable. The ultrasound and/or shockwave source 320 has a focal spot 322 which moves together with the source. In an embodiment the distance of the focal spot to the source may be modified.

To describe the relative movement, a cartesian coordinate system 280 may be used. There is an x-axis 281, which may be a longitudinal axis through the center of the table. Furthermore, there is a y-axis 282 orthogonal to the x-axis and in the plane of the table surface. A z-axis 283 is orthogonal to the plane of the table surface and therefore orthogonal to the x-axis and the y-axis. There may also be a first rotation 284 around the x-axis, and a second rotation 285 around the y-axis. A positive rotation may be a clockwise rotation in a view along a positive axis.

For control of the movement of the ultrasound and/or shockwave source 320 relative to the patient table 200, a control panel 400 may be provided.

In Figure 2, a control panel 400 is shown in detail. The control panel 400 may also be integrated in a monitor or be part of a personal computer. It may also be integrated into a therapy system or be integrated or attached to a patient table 200. The control panel 400 may include a display 420, which may have a touchscreen and/or control buttons 430 and/or combined control buttons and indicators 440. The control panel 400 further includes a control device 410 which may be a five-axis input device or may be a six-axis input device. Possible input axes are shown be the coordinate system 480. The control device 410 may accept input in three axes: an x-axis (481), a y-axis (482) and a z-axis (483) by pushing or pulling in the direction of the axes. The control device 410 may further accept input of two rotations: around the x-axis (484) and around the y-axis (485) by tilting along the rotations. The control device may react on a force and/ or a movement and output a control signal about a force applied along a certain axis or a combination of axes. In another embodiment, the control device 410 may be enabled to perform a sliding movement upon pressure.

A control unit 500 may for example be incorporated into the stand 200. This control unit may cause the positioning device 240 to perform a movement along the signaled axes or directions.

If - as mentioned above - the movement of the ultrasound and/or shockwave source 320 relative to the patient table 200 follows the force to the control device 410 or the deflection of the control device 410, handling of the shockwave therapy system may be very intuitive.

In case of a device with six degrees of freedom, rotation about the z-axis may be used for additional control of at least an additional function. Such a function may be movement of the focus spot towards or away from the ultrasound and/or shockwave source, preferably along a center axis of the source. This may be done by adjusting the focal width of the source and/or by performing a combined movement on at least one of the x-axis, y-axis and z-axis. By this way the source may be displaced along an axis through the center of the source.

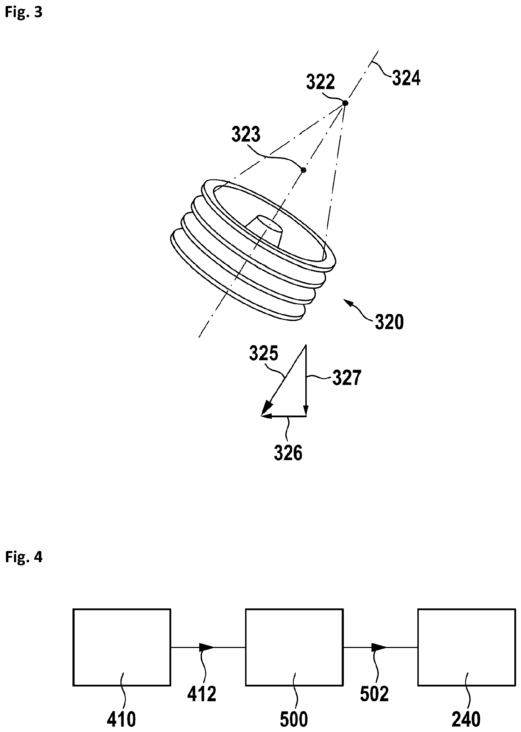

Figure 3 shows a focal spot translation by displacing the source in three axes. In this figure only a two-axis translation is show in the drawing plane, but in a real therapy system, a displacement may be made in one, two or three axes based upon the location of the displacement vector.

The figure shows an ultrasound and/or shockwave source 320 having a focal spot 322 which may be on the center axis 324 of the source, which may also be called the focal axis. Now, the focal spot should be moved along the center axis to a new location 320, which may be closer to the target area. This displacement requires a displacement vector 325. This displacement vector has an x-component 326 and a z-component 327. There may also be a y-component which is not shown in this 2-dimensional example.

The operator of the therapy system may set the control device 410, for example by a rotation about the z-axis to start a relative movement of the source and /or the table for the displacement vector 325 along the center axis. In response to this setting, the control panel 400 and/or the control unit 500 may calculate the displacement vector components in a cartesian coordinate system or a coordinate system adapted to the therapy system, such that the system can perform the relative movement about the displacement vector 325.

In figure 4, a functional block diagram is shown. The control device 410 generates output signals 412 which are coupled to the control unit 500. The control unit 500 generates control signals 502 to control the positioning device 240 for movements resulting in modification of the spatial relationship and orientation between the source 320 and the patient table 200.

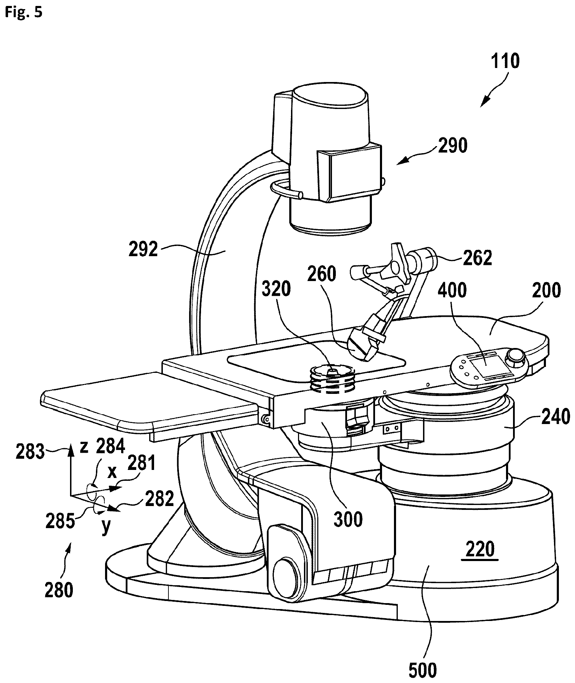

In figure 5, a modified embodiment is shown. Here, an additional X-ray device 290, acting a targeting device, is provided. It may have an X-ray tube at a C-arm 292 and may be used for localizing a stone (a target area) in the body of a patient additionally or alternatively to the ultrasound transducer 260. The other components are basically the same as described in the previous embodiment.

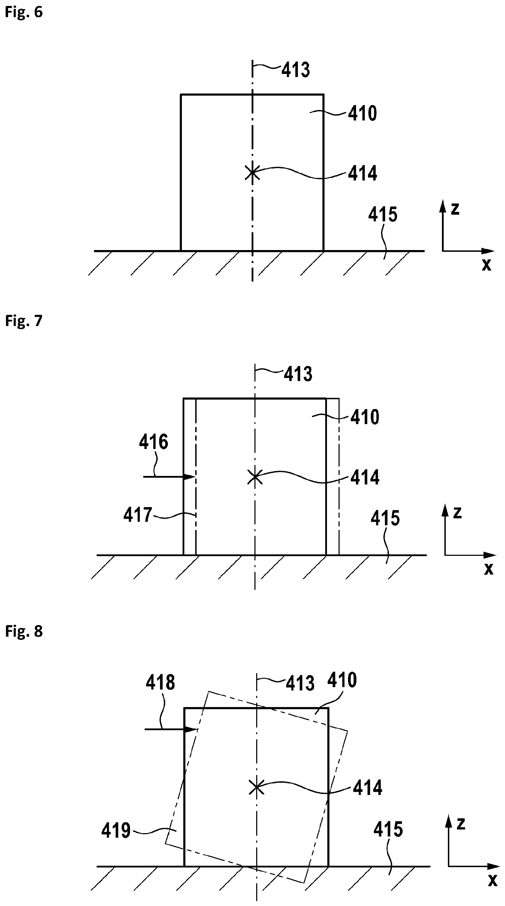

Figure 6 shows a schematic drawing of a control device 410, which may have a cylindrical shape. A control device 410 may be on a baseplane 415 and may have a center of suspension 414 which may be above the baseplane 415 to allow for tilting and displacement. The baseplane may be a baseplate and/or a part of the control panel 400.

Figure 7 shows a displaced device where a parallel displacement force 416 is applied to the control device 410 parallel to the baseplane 415 at the same height above the baseplane 415 as the center of suspension 414. This force may result in a displacement 417 of the control device parallel to the baseplane 415.

Figure 8 shows a tilted device, where a tilting force 418 is applied to the control device 410 at a height above the center of suspension 414. This force may result in tilting 419 or rotation of the control device with respect to the baseplane 415.

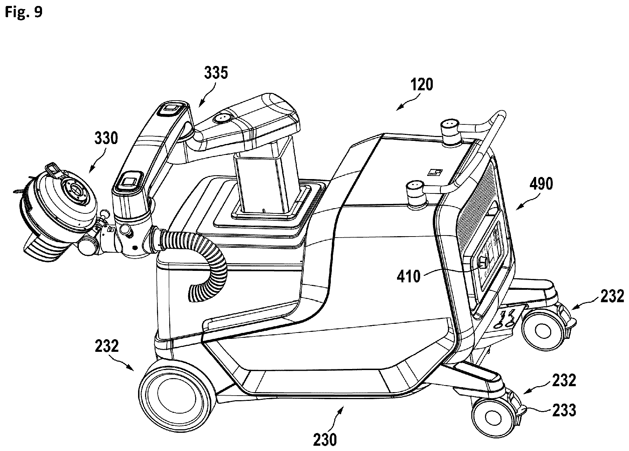

In figure 9, a further embodiment is shown. Here, a base body 230 is based on wheels 232, such that it can be moved. The wheels may be locked by locking means 233 such that a stable positional relation between the patient table (not shown in this figure) and the base body can be established. Here, the patient table may be standard table or the table of another device like an X-ray device or a CT scanner. The patient table must also be lockable in its position on the floor. The ultrasound and/or shockwave source 330 may be movable by an arm 335. A control device 410 may be on removable control panel 490.

### List of reference numerals

- 100: ultrasound and/or shockwave therapy system
- 110: ultrasound and/or shockwave therapy system with x-ray imaging
- 120: mobile ultrasound and/or shockwave therapy system 200 patient table
- 220: stand
- 230: base body
- 232: wheels
- 233: wheel locking means
- 240: positioning device
- 260: ultrasound transducer
- 262: arm
- 280: first cartesian coordinate system
- 281: x-axis
- 282: y-axis
- 283: z-axis
- 284: tilt around the x-axis
- 285: tilt around the y-axis
- 290: X-ray device
- 292: C-arm
- 300: ultrasound and/or shockwave device
- 320: ultrasound and/or shockwave source
- 322: focal spot
- 323: focal spot after movement
- 324: source center axis
- 325: source displacement vector
- 326: source displacement vector x component
- 327: source displacement vector z component
- 330: ultrasound and/or shockwave source
- 400: control panel
- 410: control device
- 412: output signal
- 413: center axis of control device
- 414: center of suspension
- 415: baseplane of control device
- 416: parallel displacement force
- 417: displaced control device
- 418: tilting force
- 419: tilted control device
- 420: display
- 430: control buttons
- 440: combined control buttons and indicators
- 480: second cartesian coordinate system
- 481: x-axis
- 482: y-axis
- 483: z-axis
- 484: tilt around the x-axis
- 485: tilt around the y-axis
- 486: rotation around the y-axis
- 490: removable control panel
- 500: control unit
- 502: control signals

## Claims

1. An ultrasound and/or shockwave therapy system (100) comprising at least:
an ultrasound and/or shockwave source (320),
a patient table (200),
a positioning device (240) between the source and the patient table,
a control device (410), which is configured to be touched or moved by an operator, and
a control unit (500) which is coupled to receive the output signals from the control device (410) and to control the positioning device (240) to modify the spatial relationship and orientation between the source (320) and the patient table (200) according to the signals from the input device **characterized in, that**
the positioning device (240) is configured to modify the spatial relationship and orientation between the source (320) and the patient table (200) in at least five degrees of freedom,
the control device (410), is configured to sense forces and/or movements in six degrees of freedom and to generate output signals (412) according to the sensed forces or movements, wherein
rotation about the z-axis of the control device (410) is used to control movement of the focus spot of the ultrasound and/or shockwave source (320) towards or away from the ultrasound and/or shock-wave source (320).

2. A therapy system (100) according to claim 1,
**characterized in, that**
the six degrees of freedom comprise within a cartesian coordinate system:
three degrees of translation on three orthogonal axes and
at least two degrees of rotation or tilting.

3. A therapy system (100) according to any of the previous claims,
**characterized in, that**
the control unit (500) generates control signals (502) to the positioning device (240) to move the source (320) along a center axis (324) of the source.

4. A therapy system (100) according to claim 3,
**characterized in, that**
the sixth degrees of freedom of the control device (410) is a force and/or movement perpendicular to the surface of control panel (400) to which the control device (410) is attached.

5. A therapy system (100) according to any of the previous claims,
**characterized in, that**
the control unit (500) is configured to set the positioning speed of the positioning device (240) according to the output signals (412) of the control device (410).

6. A therapy system (100) according to claim 5,
**characterized in, that**
the control unit (500) is configured to set the positioning speed of the positioning device (240) according to the output signals (412) of the control device (410) based on a linear function or based on an exponential function.

7. A therapy system (100) according to any of the previous claims,
**characterized in, that**
a first cartesian coordinate system is defined by a first x-axis (281) being a longitudinal axis through the center of a surface of the patient table (200), a first y-axis (282) orthogonal to the first x-axis (281) on the surface of the patient table (200), and a first z-axis (283) orthogonal to the first x-axis (281) and the first y-axis (282) pointing upward from the surface of the patient table (200), and
a second cartesian coordinate system is defined by a second x-axis (481) being a longitudinal axis parallel to a baseplane (415) of the control device (410), a second y-axis (482) orthogonal to the second x-axis (481) and parallel to the baseplane (415), and a second z-axis (483) orthogonal to the second x-axis (481) and the second y-axis (482) pointing upward from the baseplane (415) and preferably through a center axis (413) of the control device.

8. A therapy system (100) according to claim 7,
**characterized in, that**
the control unit (500) is configured to give control signals (502) to the positioning device (240) dependent of forces and/or movements of the control device (410) as follows:
- forces and/or movements of control device (410) - x-axis (481) results in control signal (502) for x-axis (281);
- forces and/or movements of control device (410) - y-axis (482) results in control signal (502) for y-axis (282);
- forces and/or movements of control device (410) - z-axis (483) results in control signal (502) for z-axis (283);
- forces and/or movements of control device (410) - tilt around x-axis (481) results in control signal (502) for tilt around x-axis (281);
- forces and/or movements of control device (410) - tilt around y-axis (482) results in control signal (502) for tilt around y-axis (282);

9. A therapy system (100) according to claim 8,
**characterized in, that**
the control unit (500) is configured to give control signals (502) to the positioning device (240) dependent of forces and/or movements of the control device (410) as follows:
- forces and/or movements of control device (410) - twist around z-axis (482) results in control signal (502) for a movement on the source center axis (324).

## Patentansprüche

1. Ein Ultraschall- und/oder Stoßwellentherapiesystem (100), umfassend mindestens:
eine Ultraschall- und/oder Stoßwellenquelle (320),
einen Patiententisch (200),
eine Positionierungsvorrichtung (240) zwischen der Quelle und dem Patiententisch,
eine Steuervorrichtung (410), die dazu konfiguriert ist, von einem Bediener berührt oder bewegt zu werden, und
eine Steuervorrichtung (500), die gekoppelt ist, um die Ausgabesignale von der Steuervorrichtung (410) zu empfangen und die Positionierungsvorrichtung (240) zu steuern, um die räumliche Beziehung und Ausrichtung zwischen der Quelle (320) und dem Patiententisch (200) gemäß den Signalen von der Eingabevorrichtung zu modifizieren
**dadurch gekennzeichnet, dass**
die Positionierungsvorrichtung (240) konfiguriert ist, um die räumliche Beziehung und Ausrichtung zwischen der Quelle (320) und dem Patiententisch (200) in mindestens fünf Freiheitsgraden zu modifizieren,
die Steuervorrichtung (410) konfiguriert ist, um Kräfte und/oder Bewegungen in sechs Freiheitsgraden zu erfassen und Ausgabesignale (412) gemäß den erfassten Kräften oder Bewegungen zu erzeugen, wobei
eine Rotation um die z-Achse der Steuervorrichtung (410) verwendet wird, um eine Bewegung des Brennpunktes der Ultraschall- und/oder Schockwellenquelle (320) hin zu oder weg von der Ultraschall- und/oder Schockwellenquelle (320) zu steuern.

2. Ein Therapiesystem (100) nach Anspruch 1,
**dadurch gekennzeichnet, dass**
die sechs Freiheitsgrade innerhalb eines kartesischen Koordinatensystems umfassen:
drei Translationsgrade auf drei orthogonalen Achsen und
mindestens zwei Rotations- oder Neigungsgrade.

3. Ein Therapiesystem (100) gemäß einem der vorhergehenden Ansprüche,
**dadurch gekennzeichnet, dass**
die Steuervorrichtung (500) Steuersignale (502) an die Positioniervorrichtung (240) erzeugt, um die Quelle (320) entlang einer Mittelachse (324) der Quelle zu bewegen.

4. Ein Therapiesystem (100) nach Anspruch 3,
**dadurch gekennzeichnet, dass**
die sechste Freiheitsgrad der Steuervorrichtung (410) eine Kraft und/oder Bewegung senkrecht zur Oberfläche des Bedienfelds (400) ist, an dem die Steuervorrichtung (410) angebracht ist.

5. Ein Therapiesystem (100) gemäß einem der vorhergehenden Ansprüche,
**dadurch gekennzeichnet, dass**
die Steuervorrichtung (500) konfiguriert ist, um die Positioniergeschwindigkeit der Positioniervorrichtung (240) gemäß den Ausgabesignalen (412) der Steuervorrichtung (410) einzustellen.

6. Ein Therapiesystem (100) nach Anspruch 5,
**dadurch gekennzeichnet, dass**
die Steuervorrichtung (500) konfiguriert ist, um die Positioniergeschwindigkeit der Positioniervorrichtung (240) gemäß den Ausgabesignalen (412) der Steuervorrichtung (410) basierend auf einer linearen Funktion oder basierend auf einer Exponentialfunktion einzustellen.

7. Ein Therapiesystem (100) nach einem der vorhergehenden Ansprüche,
**dadurch gekennzeichnet, dass**
ein erstes kartesisches Koordinatensystem definiert ist durch eine erste x-Achse (281), die eine Längsachse durch die Mitte einer Oberfläche des Patiententisches (200) ist, eine erste y-Achse (282) orthogonal zur ersten x-Achse (281) auf der Oberfläche des Patiententisches (200), und eine erste z-Achse (283) orthogonal zur ersten x-Achse (281) und zur ersten y-Achse (282), die von der Oberfläche des Patiententisches nach oben zeigt (200) und ein zweites kartesisches Koordinatensystem definiert ist durch eine zweite x-Achse (481), die eine Längsachse parallel zu einer Basisebene (415) der Steuervorrichtung (410) ist, eine zweite y-Achse (482) orthogonal zur zweiten x-Achse (481) und parallel zur Basisebene (415), und eine zweite z-Achse (483) orthogonal zur zweiten x-Achse (481) und zur zweiten y-Achse (482), die von der Basisebene (415) nach oben und vorzugsweise durch eine Mittelachse (413) der Steuereinrichtung zeigt.

8. Ein Therapiesystem (100) nach Anspruch 7,
**dadurch gekennzeichnet, dass**
die Steuervorrichtung (500) konfiguriert ist, um Steuersignale (502) an die Positioniervorrichtung (240) in Abhängigkeit von Kräften und/oder Bewegungen der Steuervorrichtung (410) wie folgt zu geben:
- Kräfte und/oder Bewegungen der Steuereinrichtung (410) - x-Achse (481) führt zu Steuersignal (502) für x-Achse (281);
- Kräfte und/oder Bewegungen der Steuereinrichtung (410) - y-Achse (482) führt zu Steuersignal (502) für y-Achse (282);
- Kräfte und/oder Bewegungen der Steuereinrichtung (410) - z-Achse (483) führt zu Steuersignal (502) für z-Achse (283);
- Kräfte und/oder Bewegungen der Steuereinrichtung (410) - Kippen um x-Achse (481) führt zu Steuersignal (502) für Kippen um x-Achse (281);
- Kräfte und/oder Bewegungen der Steuereinrichtung (410) - Kippen um die y-Achse (482) führt zu Steuersignal (502) zum Kippen um die y-Achse (282);

9. Ein Therapiesystem (100) nach Anspruch 8,
**dadurch gekennzeichnet, dass**
die Steuervorrichtung (500) konfiguriert ist, um Steuersignale (502) an die Positioniervorrichtung (240) in Abhängigkeit von Kräften und/oder Bewegungen der Steuervorrichtung (410) wie folgt zu geben:
- Kräfte und/oder Bewegungen der Steuervorrichtung (410) - Drehen um die z-Achse (482) führt zu Steuersignal (502) für eine Bewegung auf der Quellen-Mittelachse (324).

## Revendications

1. Système de thérapie par ultrasons et/ou ondes de choc (100) comprenant au moins :
une source d'ultrasons et/ou d'ondes de choc (320),
une table d'examen (200),
un dispositif de positionnement (240) entre la source et la table d'examen,
un dispositif de commande (410), qui est configuré pour être touché ou déplacé par un opérateur, et
une unité de commande (500) qui est couplée pour recevoir les signaux de sortie du dispositif de commande (410) et pour commander le dispositif de positionnement (240) afin de modifier la relation et l'orientation spatiales entre la source (320) et la table d'examen (200) selon les signaux du dispositif d'entrée
**caractérisé en ce que**
le dispositif de positionnement (240) est configuré pour modifier la relation et l'orientation spatiales entre la source (320) et la table d'examen (200) dans au moins cinq degrés de liberté,
le dispositif de commande (410) est configuré pour capter des forces et/ou des déplacements dans six degrés de liberté et pour générer des signaux de sortie (412) selon les forces ou les déplacements captés, dans lequel
une rotation autour de l'axe z du dispositif de commande (410) est utilisée pour commander un déplacement du point focal de la source d'ultrasons et/ou d'ondes de choc (320) vers ou en éloignement de la source d'ultrasons et/ou d'ondes de choc (320).

2. Système de thérapie (100) selon la revendication 1, **caractérisé en ce que**
les six degrés de liberté comprennent au sein d'un système de coordonnées cartésiennes :
trois degrés de translation sur trois axes orthogonaux et
au moins deux degrés de rotation ou d'inclinaison.

3. Système de thérapie (100) selon l'une quelconque des revendications précédentes,
**caractérisé en ce que**
l'unité de commande (500) génère des signaux de commande (502) pour le dispositif de positionnement (240) pour déplacer la source (320) selon un axe de centre (324) de la source.

4. Système de thérapie (100) selon la revendication 3,
**caractérisé en ce que**
les six degrés de liberté du dispositif de commande (410) sont une force et/ou un déplacement perpendiculaires à la surface d'un panneau de commande (400) auquel le dispositif de commande (410) est attaché.

5. Système de thérapie (100) selon l'une quelconque des revendications précédentes,
**caractérisé en ce que**
l'unité de commande (500) est configurée pour régler la vitesse de positionnement du dispositif de positionnement (240) selon les signaux de sortie (412) du dispositif de commande (410).

6. Système de thérapie (100) selon la revendication 5,
**caractérisé en ce que**
l'unité de commande (500) est configurée pour régler la vitesse de positionnement du dispositif de positionnement (240) selon les signaux de sortie (412) du dispositif de commande (410) d'après une fonction linéaire ou d'après une fonction exponentielle.

7. Système de thérapie (100) selon l'une quelconque des revendications précédentes,
**caractérisé en ce que**
un premier système de coordonnées cartésiennes est défini par un premier axe x (281) qui est un axe longitudinal à travers le centre d'une surface de la table d'examen (200), un premier axe y (282) orthogonal au premier axe x (281) sur la surface de la table d'examen (200), et un premier axe z (283) orthogonal au premier axe x (281) et au premier axe y (282) pointant vers le haut depuis la surface de la table d'examen (200), et
un second système de coordonnées cartésiennes est défini par un second axe x (481) qui est un axe longitudinal parallèle à un plan de base (415) du dispositif de commande (410), un second axe y (482) orthogonal au second axe x (481) et parallèle au plan de base (415), et un second axe z (483) orthogonal au second axe x (481) et au second axe y (482) pointant vers le haut depuis le plan de base (415) et de préférence à travers un axe de centre (413) du dispositif de commande.

8. Système de thérapie (100) selon la revendication 7,
**caractérisé en ce que**
l'unité de commande (500) est configurée pour donner des signaux de commande (502) au dispositif de positionnement (240) en fonction des forces et/ou des déplacements du dispositif de commande (410) comme suit :
- des forces et/ou des déplacements du dispositif de commande (410) - axe x (481) donnent lieu à un signal de commande (502) pour l'axe x (281) ;
- des forces et/ou des déplacements du dispositif de commande (410) - axe y (482) donnent lieu à un signal de commande (502) pour l'axe y (282) ;
- des forces et/ou des déplacements du dispositif de commande (410) - axe z (483) donnent lieu à un signal de commande (502) pour l'axe z (283) ;
- des forces et/ou des déplacements du dispositif de commande (410) - inclinaison autour de l'axe x (481) donnent lieu à un signal de commande (502) pour une inclinaison autour de l'axe x (281) ;
- des forces et/ou des déplacements du dispositif de commande (410) - inclinaison autour de l'axe y (482) donnent lieu à un signal de commande (502) pour une inclinaison autour de l'axe y (282).

9. Système de thérapie (100) selon la revendication 8,
**caractérisé en ce que**
l'unité de commande (500) est configurée pour donner des signaux de commande (502) au dispositif de positionnement (240) en fonction des forces et/ou des déplacements du dispositif de commande (410) comme suit :
- des forces et/ou des déplacements du dispositif de commande (410) - torsion autour de l'axe z (482) donnent lieu à un signal de commande (502) pour un déplacement sur l'axe de centre (324) de la source.
